# EUROPEAN PATENT SPECIFICATION

(11) **EP 0 546 846 B1**
(45) Date of publication and mention of the grant of the patent: **22.01.1997**
(21) Application number: 92311328.6
(22) Date of filing: 11.12.1992
(51) Int. Cl.: A61K 31/135, A61K 9/16, A61K 9/50

(54) **Programmed-release pharmaceutical compositions**
Arzneimittel mit gesteuerter Wirkstoffabgabe
Compositions pharmaceutiques à libération programmée

(30) Priority: 11.12.1991 IT MI913318
(43) Date of publication of application: 16.06.1993
(73) Proprietor: THERAPICON SRL, I-20146 Milan (IT)
(72) Inventor: Veronesi, Paolo Alberto, 20146 Milano (IT); Veronesi, Anna Maria, 06083 Bastia Umbra PG (IT)
(74) Representative: Hardisty, David Robert

(56) References cited:
- EP-A- 0 125 634
- EP-A- 0 208 144
- DE-A- 3 943 242

## Description

The invention relates to programmed-release compositions of ambroxol hydrochloride and a process for preparing microgranules of the same.

Ambroxol hydrochloride is known to be a pharmacologically active substance and a valuable lung alveoles surfactant protecting agent, very useful in the prevention of chronic bronchial exacerbations and sarcoidosis. However, it possesses a relatively short biological half-life and therefore it has been necessary to administer it at least three times a day in order that it exhibits its full action.

It is desirable to decrease the number of administrations of ambroxol hydrochloride. This will not only reduce the burden on the patient but wil also increase his compliance, thus providing greater therapeutic effects.

Some of the techniques, which have been proposed for preparing slow-release pharmaceutical compositions containing ambroxol hydrochloride, are capable of retaining the concentrations of this active ingredient in the blood for a prolonged period of time, but neither the minimum quantity of ambroxol hydrochloride to be released from the preparation "in vitro" after fixed intervals (programmed liberation profile with the indication of the minimum quantity to be released at the fixed intervals) nor the influence and possible variations of the preparation during the ageing period (stability of the programmed-release-rate of the preparation) have been considered in the previous art.

According to the invention there is provided a process for preparing programmed-release microgranules comprising ambroxol hydrochloride, characterized in that said process includes the following steps:
providing cores of from 0.3 to 1.2 mm in size containing substantially no pharmacologically active amount of ambroxol hydrochloride, coating said cores with multiple microlayers of a composition comprising a mixture of particles of ambroxol hydrochloride from 1 to 150 microns in size and a delayed release film-coating component wherein said coating component is such that the microgranules have a liberation rate of ambroxol hydrochloride as follows:

| | |
|---|---|
| 1st hour | 20-40% |
| 8th hour | 55-75% |
| 16th hour | >75%. |

The invention further provides an ambroxol HCl pharmaceutical microgranule dosage form which releases ambroxol HCl according to a predetermined programme, comprising a plurality of core microgranules of a variety of particle sizes ranging from 0.3 to 1.2 mm which contain substantially no pharmacologically active ambroxol hydrochloride, said core microgranules being coated with multiple microlayers of (a) micronized ambroxol hydrochloride and (b) delayed release film material, such that said coated microgranules will deliver the ambroxol hydrochloride content thereof, in vivo, according to the following release rate profile:

| | |
|---|---|
| 1st hour | 20% to 40% |
| 8th hour | 55% to 75% |
| 16th hour | > 75%. |

Preferably the cores contain substantially no pharmacologically active amount of ambroxol hydrochloride and more preferably contain none at all. Preferably they are made from starch, sugar or a combination of both. Cores of the required size may be obtained by known means; preferably they are selected by sieving them through a sieve having openings from 0.3 mm to 1.2 mm.

Ambroxol hydrochloride is preferably associated with talc or other supports e.g aluminium or magnesium silicates or lactose. Ambroxol hydrochloride particles of the preferred size may be obtained by known means but preferably the ambroxol hydrochloride with or without associated support is micronized.

Preferably the film-coating component is comprised of polyvinylpyrrolidone, polyvinylpyrrolidone associated with shellac, shellac, acrylic resin (e.g brand Eudragit, Röhm Pharma, Germany) hydroxypropyl methylcellulose, ethylcellulose or derivatives of the same or mixtures of the above substances. The coating component may be dissolved and/or suspended in solution. Solvents may be any known appropriate solvent but preferably it is an aqueous or an organic solvent including isopropanol or other alcohol, acetone or a mixture of aqueous and organic solvents. As will be known to the skilled man it will be necessary that the coating component be selected such that it will allow ambroxol hydrochloride to be liberated as required. The coating component should comprise at least 50% of one or more compounds which are insoluble in acqueous medium including shellac, acrylic resin or other compound or mixture. Preferably this percentage is from 50 to 90% and more preferably it is from 70 to 85%. The coating component may also include polyvinylpyrrolidones, hydroxypropyl methylcellulose, ethylcellulose or derivatives of the same or other acqueous soluble film forming compounds or mixtures of compounds. Most preferably that percentage of the coating component which is not acqueous insoluble compound is acqueous soluble compound.

The ambroxol hydrochloride may be incorporated with the coating component by any means known to the skilled man. Such means may also provide means of applying the composition to the cores. One example of such means comprises spraying systems which may be with air or without air. Such spraying means may be used to spray both the ambroxol hydrochloride and coating component onto the cores. The ambroxol hydrochloride particles may be mixed with the coating component prior to spraying however, they are preferably applied simultaneously as different sprays. This separate but simultaneous spraying preferably provides decreased drying time compared to that where the composition is mixed prior to application.

The composition provided around the core may comprise one or more layers.

The concentration of ambroxol hydrochloride in the microgranules is preferably from 100 to 500 mg per gram.

Talc may also be added to the coating component.

An outer coating of a coating component may be applied to the microgranules.

The microgranules produced according to the process of the invention may be inserted into a capsule such that they are suitable for administration to a patient.

Preferably the microgranules produced by the process of this invention will slowly release almost all their content of the active substance ambroxol hydrochloride over a prolonged period of time, and more preferably over about 16 hours in order to prolong the plasmatic levels for in excess of 24 hours.

The manufactured batches may be submitted to a dissolution test to determine the liberation rate profile by the means of the equipment recommended by U.S.P. XXII, Model 1 at 100 rotation per minute, by using 900 ml of water as immersion liquid.

The programmed-release microgranules of ambroxol hydrochloride may be then easily administered at the recommended doses, as they are more conveniently packed in unitary daily doses in primary containers made from hard gelatine.

### EXAMPLE 1

Cores comprising sugar and starch are prepared starting from 500 g of sugar and from 500 g of an aqueous solution of sugar and starch (1:1).

A 500 g solution of sugar and starch was injected in a rotating pan containing 500 g of sugar. Non-pharmacologically active cores of almost sphered form are obtained. The cores are dried for 24 hours at 50°C. The cores are then sieved to obtain cores from 0.3 to 1.2 mm.

190 g of micronized ambroxol hydrochloride and starch (98:2) having a particle size of about 10 microns is sprayed as a dry powder onto 500 g of cores. Simultaneously, about 100 g of a 20% solution of shellac (80%) and polyvinylpyrrolidone (20%) in isopropanol is sprayed onto the cores such that it combines with the ambroxol hydrochloride powder and provides a coating around the cores. The spraying may take place in intervals so that a portion of the composition may partly or fully dry before the remainder of the composition is applied.

2 batches of the microgranules were produced.
1) the size of the micronized particles ambroxol hydrochloride which were used are from 1 to 150 microns. These were tested prior to use;
2) the quantity of micronized ambroxol hydrochloride to be incorporated was from 100 to 500 mg/g of the final product;
3) the quantity and the proportions of shellac and polyvinylpyrrolidone is varied according to the needs;
4) for a better modulation of the programmed-release profile, shellac may be substituted or added with another film-coating agent.

### EXAMPLE 2

### Liberation rates

Samples of the products of the process referred to in Example 1 were tested according to the U.S.P. XXII system, Model 1 at 100 rotation per minute, by using 900 ml of water as immersion liquid, and the following results were obtained:

| | Specifications | Obtained liberation profile |
|---|---|---|
| | | |

| Product from batch 1 of Example 1 | | |
|---|---|---|
| - 1st hour | 20-40% | 29% |
| - 8th hour | 55-75% | 72% |
| -16th hour | 75% | 89% |

| Product from batch 2 of Example 1 | | |
|---|---|---|
| - 1st hour | 20-40% | 28% |
| - 8th hour | 55-75% | 72% |
| -16th hour | > 75% | 91% |

### EXAMPLE 3

Stability tests were performed to confirm the liberation profile and other basic parameteres of the grogrammed-release microgranules of the two batches of Example 1.

Five samples of 5 grams were taken from each of the two batches of Example 1 and were packed in amber glass bottles and the plastic screw-caps were attached. The bottles were then stored at 25° and 50°C respectively for a consecutive period of 5 years and tested at the end of each interval of one year, with the following results:

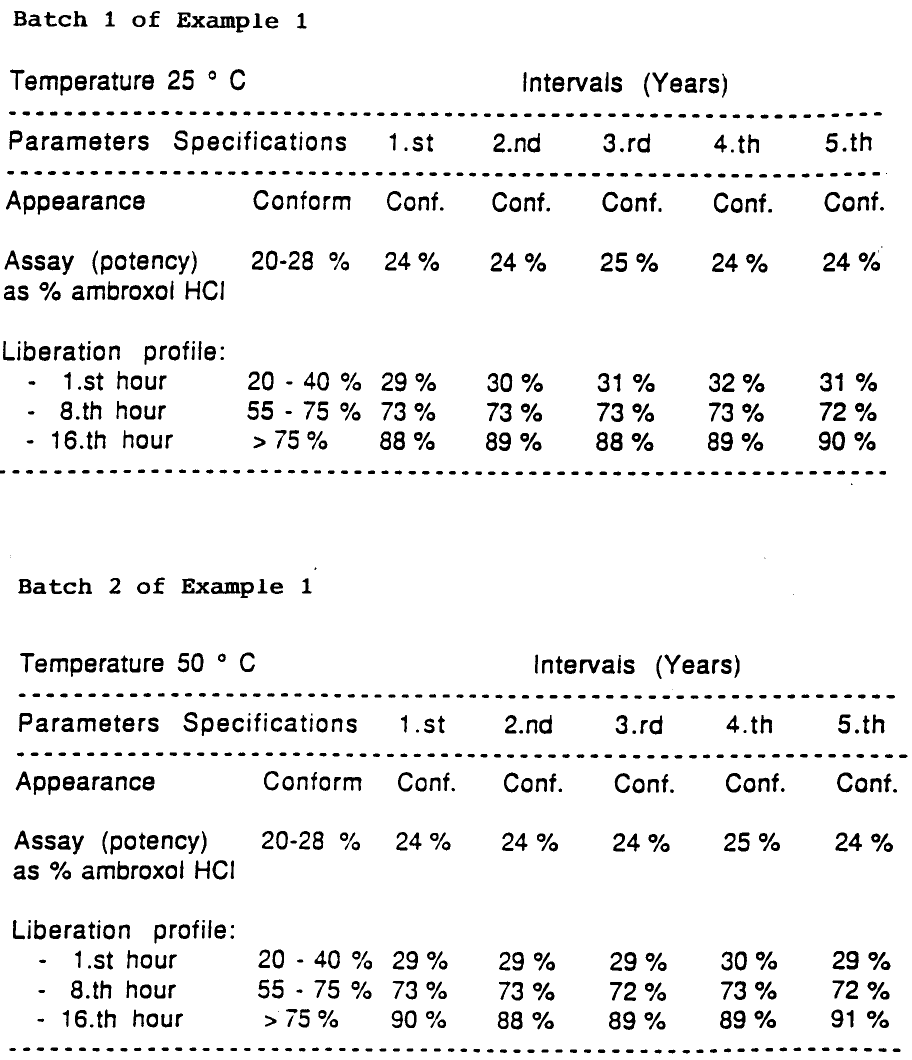

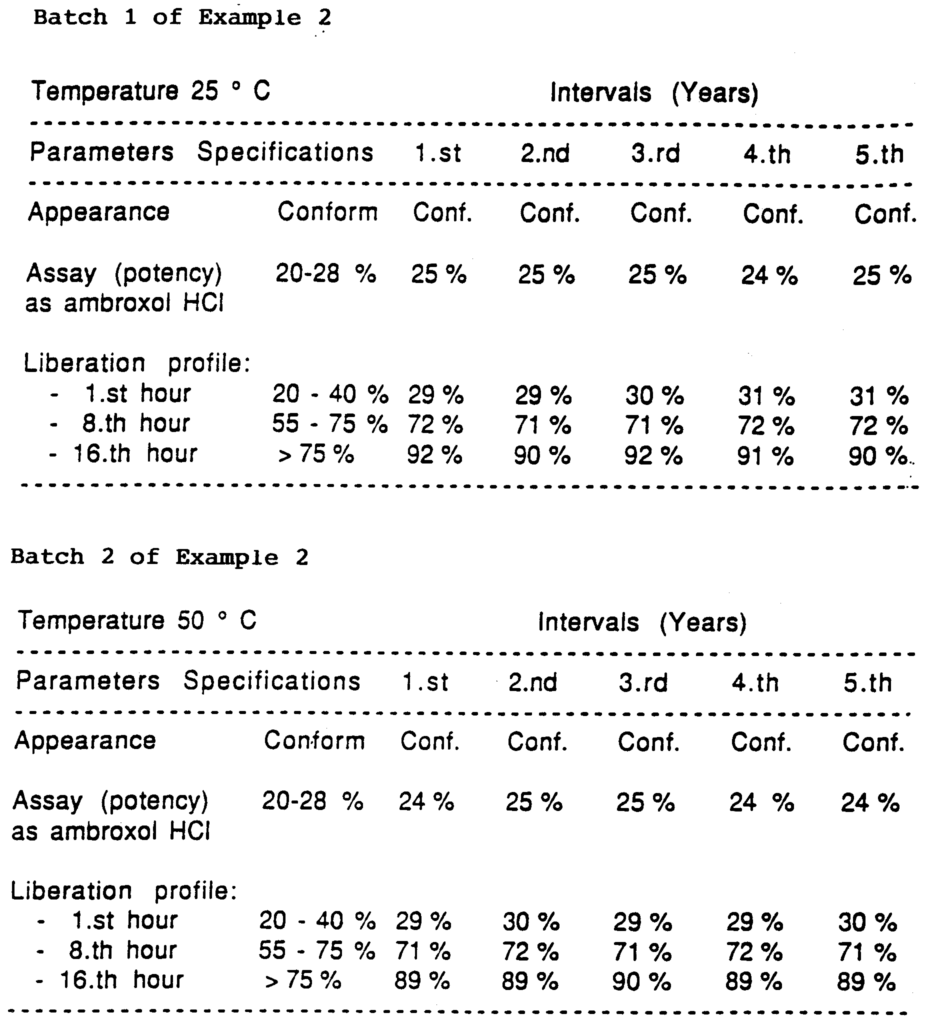

### EXAMPLE 4

Plasmatic concentrations " in vivo " following the administration of a single dose of the product obtained from batch 1 of Example 1.

A single hard gelatine capsule containing about 310 mg of microgranules, corresponding to about 75 mg of ambroxol hydrochloride, of the product obtained from batch 1 of Example 1, was administered to each of 12 healthy volunteers, in order to determine the resulting plasmatic levels of the active ingredient.

The following results of the plasmatic concentrations were obtained (results expressed as ambroxol, µg/ml) :

| Subjects | Time after single dose administration (in hours) | | | | | | | | | |
|---|---|---|---|---|---|---|---|---|---|---|
| N°. | 00 | 01 | 02 | 04 | 08 | 012 | 16 | 24 | 32 | 36 |
| 01 | 0.0 | 16.4 | 110.6 | 175.1 | 145.1 | 100.4 | 50.1 | 34.7 | 29.4 | 15.1 |
| 02 | 0.0 | 10.7 | 75.7 | 95.7 | 118.7 | 67.5 | 55.5 | 39.5 | 30.7 | N.D. |
| 03 | 0.0 | 14.9 | 131.9 | 141.3 | 90.9 | 81.5 | 59.9 | 40.9 | 33.9 | 19.0 |
| 04 | 0.0 | 17.0 | 121.9 | 122.9 | 103.5 | 85.3 | 55.3 | 49.7 | 33.0 | 18.4 |
| 05 | 0.0 | 25.2 | 155.1 | 229.1 | 160.4 | 110.8 | 70.4 | 66.0 | 56.4 | N.D. |
| 06 | 0.0 | 12.1 | 92.0 | 191.4 | 138.9 | 118.8 | 71.5 | 65.1 | 52.2 | 21.4 |
| 07 | 0.0 | N.D. | 85.6 | 167.2 | 124.7 | 100.0 | 48.2 | 37.7 | 29.5 | N.D. |
| 08 | 0.0 | 12.8 | 100.5 | 242.7 | 153.7 | 97.5 | 54.1 | 41.9 | 44.7 | 20.4 |
| 09 | 0.0 | 31.5 | 87.2 | 182.1 | 127.8 | 114.3 | 82.9 | 50.8 | 43.8 | 18.3 |
| 10 | 0.0 | 23.6 | 137.4 | 158.7 | 110.3 | 81.9 | 50.9 | 43.3 | 37.8 | N.D. |
| 11 | 0.0 | 18.7 | 140.0 | 150.2 | 130.1 | 80.2 | 59.4 | 33.9 | 23.7 | N.D. |
| 12 | 0.0 | 20.6 | 111.5 | 139.4 | 116.0 | 77.7 | 73.7 | 61.2 | 50.1 | 33.4 |
| M | 0.0 | 16.9 | 112.4 | 166.3 | 126.6 | 92.9 | 61.0 | 47.0 | 38.7 | 12.1 |
| S.D. | 0.0 | 8.04 | 25.12 | 41.85 | 20.52 | 16.30 | 11.02 | 11.49 | 10.46 | 11.58 |
| S.E. (+/-) | 0.0 | 2.32 | 7.25 | 12.08 | 5.92 | 4.70 | 3.18 | 3.31 | 3.01 | 3.34 |
| M = Mean SD = Standard deviation SE (+/-) = standard error | | | | | | | | | | |

The pharmacokinetic parameters are calculated to be as follows:

| Parameter | Obtained values |
|---|---|
| AUC 0 - 24 (µg/hour/ml ⁻¹) | 2815.42 |
| Ke (hour ⁻¹) | 0.1038 |
| T max (hour) | 4.15 |
| C max (hour) | 1564.36 |
| t 1/2 α phase (hour) | 2.77 |
| t 1/2 β phase (hour) | 14.41 |

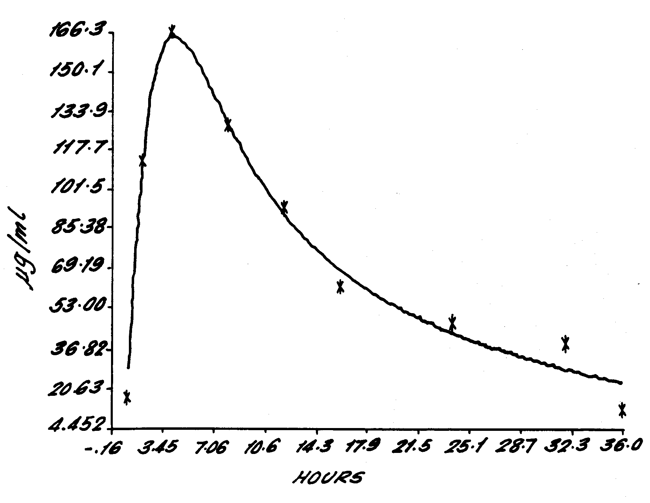

A graph of the mean plasma concentrations of ambroxol hydrochloride following the administration in single dose to the 12 healthy volunteers of a hard gelatine capsule containing programmed-release microgranules dosed at 75 mg of ambroxol hydrochloride of the batch 1 of Example 1 of the invention, has been plotted and is shown in the drawing.

## Claims

1. A process for preparing programmed release microgranules comprising ambroxol hydrochloride, characterized in that said process includes the following steps:
providing cores of from 0.3 to 1.2 mm in size containing substantially no pharmacologically active amount of ambroxol hydrochloride,
coating said cores with multiple microlayers of a composition comprising a mixture of particles of ambroxol hydrochloride from 1 to 150 microns in size and a delayed release film-coating component wherein said coating component is such that the microgranules have a liberation rate of ambroxol hydrochloride as follows:
| | |
|---|---|
| 1st hour | 20-40% |
| 8th hour | 55-75% |
| 16th hour | >75% |

2. A process according to claim 1 wherein the cores comprise sucrose, starch or a combination of sucrose and starch.

3. A process according to either claim 1 or claim 2, wherein at least 50% of the coating component comprises one or more film forming compounds which are insoluble in aqueous medium including shellac, acrylic resin or a mixture of such compounds.

4. A process according to claim 3, wherein the coating component comprises from 50 to 90% of the said insoluble film-forming compound including shellac, acrylic resin or a mixture of such compounds.

5. A process according to claim 4, wherein the coating component comprises from 70 to 85% of the said insoluble film-forming compound including shellac, acrylic resin or a mixture of such compounds.

6. A process according to any one of claims 3, 4 or 5 wherein the remainder of the coating component comprises an acqueous soluble compound including polyvinylpyrrolidone, hydroxypropyl methyl cellulose, ethylcellulose or derivatives thereof.

7. A process according to any preceding claim wherein the coating component comprises from 75 to 80% acqueous insoluble film-forming compound including shellac and from 15 to 25% aqueous soluble film-forming compound including polyvinylpyrrolidone.

8. A process according to any previous claim characterised in that the microgranules including ambroxol hydrochloride in the amount of from 100 to 500 mg/g of microgranules.

9. A process according to any previous claim characterized in that the size of the microgranuies is from 0.6 to 1.5 mm.

10. A process according to any previous claim wherein the microgranules are coated with multiple microlayers of ambroxol HCl and film-coating component.

11. An ambroxol HCl pharmaceutical microgranule dosage form which releases ambroxol HCl according to a predetermined programme, comprising a plurality of core microgranules of a variety of particle sizes ranging from 0.3 to 1.2 mm which contain substantially no pharmacologically active ambroxol hydrochloride, said core microgranules being coated with multiple microlayers of (a) micronized ambroxol hydrochloride and (b) delayed release film material, such that said coated microgranules will deliver the ambroxol hydrochloride content thereof, in vivo, according to the following release rate profile:
| | |
|---|---|
| 1st hour | 20% to 40% |
| 8th hour | 55% to 75% |
| 16th hour | > 75% |

12. A programmed release dosage form as defined by Claim 11, comprising from 100 to 500 mg of micronized ambroxol HCl per gram of said inert core microgranules.

13. A programmed released dosage form as claimed in Claim 11 or Claim 12 wherein said inert core microgranules comprise sugar and starch.

14. A programmed release dosage form as claimed in any one of Claims 11 to 13 wherein said delayed release film material comprises shellac, polyvinylpyrrolidone or polyvinylpyrrolidone/shellac admixture.

15. A programmed release dosage form as claimed in any one of Claims 11 to 14 wherein said external microlayer of delayed release film material comprises shellac, an acrylic polymer, hydroxypropyl cellulose, ethyl cellulose or mixture thereof.

16. A programmed release dosage form as claimed in any one of Claims 11 to 15 wherein said multiple microlayers of micronized ambroxol hydrochloride further comprise talc, aluminum or magnesium silicate, lactose or starch.

17. A programmed release dosage form as claimed in any one of Claims 11 to 16 comprising an outer coating of coating component said coated microgranules having particle sizes ranging from 0.6 to 1.5 mm.

18. A programmed release dosage form as claimed in any one of Claims 11 to 17 adapted to deliver the ambroxol hydrochloride content thereof, in vivo, so as to maintain a therapeutically effective plasma level of ambroxol HCl for in excess of 24 hours.

19. A programmed release dosage form as claimed in any one of Claims 11 to 18 wherein said external microlayer is devoid of said micronized ambroxol hydrochloride active agent.

20. A programmed release dosage form as claimed in any one of Claims 11 to 19 confined within a hard gelatine capsule to provide a unitary dosage amount ambroxol HCl.

## Patentansprüche

1. Verfahren zur Herstellung von Mikrogranulaten mit gesteuerter Abgabe mit Ambroxolhydrochlorid, dadurch **gekennzeichnet,** daß das Verfahren die folgenden Schritte aufweist:
Herstellen von Kernen mit 0,3 bis 1,2 mm Größe, die im wesentlichen keinen pharmakologisch aktiven Anteil von Ambroxolhydrochlorid enthalten,
Beschichten der Kerne mit mehreren Mikroschichten mit einer Zusammensetzung, die eine Mischung von Partikeln von Ambroxolhydrochlorid in einer Größe von 1 bis 150 Mikron und eine Filmbeschichtungskomponente mit verzögerter Freigabe enthält, wobei die Beschichtungskomponente so ist, daß die Mikrogranulate eine Freigaberate von Ambroxolhydrochlorid wie folgt aufweisen:
| | |
|---|---|
| 1. Stunde | 20-40% |
| 8. Stunde | 55-75% |
| 16. Stunde | > 75% |

2. Verfahren nach Anspruch 1, wobei die Kerne Saccharose, Stärke oder eine Kombination von Saccharose und Stärke aufweisen.

3. Verfahren nach Anspruch 1 oder 2, wobei zumindest 50% der Beschichtungskomponente eine oder mehr filmbindende Verbindungen aufweisen, die in wässrigem Medium nicht löslich sind, einschließlich Schellack, Akrylharz oder einer Mischung solcher Verbindungen.

4. Verfahren nach Anspruch 3, wobei die Beschichtungskomponente 50-90% der nichtlöslichen filmformenden Komponente, einschließlich Schellack, Akrylharz oder einer Mischung solcher Komponenten, aufweist.

5. Verfahren nach Anspruch 4, wobei die Beschichtungskomponente 70-85% der nichtlöslichen filmformenden Komponente, einschließlich Schellack, Akrylharz oder einer Mischung solcher Komponenten, aufweist.

6. Verfahren nach einem der Ansprüche 3, 4 oder 5, wobei der Rest der Beschichtungskomponente eine wasserlösliche Verbindung einschließlich Polyvinylpyrrolidon, Hydroxypropylmethylcellulose, Ethylcellulose oder Derivate davon aufweist.

7. Verfahren nach einem der vorstehenden Ansprüche, wobei die Beschichtungselemente 75-80% nichtwasserlösliche filmformende Verbindung, einschließlich Schellack, und 15-25% wasserlösliche filmformende Komponenten, einschließlich Polyvinylpyrrolidon, aufweist.

8. Verfahren nach einem der vorstehenden Ansprüche, dadurch **gekennzeichnet,** daß die Mikrogranulate Ambroxolhydrochlorid zu einem Anteil 100-500 mg/g der Mikrogranulate enthält.

9. Verfahren nach einem der vorstehenden Ansprüche, dadurch **gekennzeichnet,** daß die Größe der Mikrogranulate 0,6-1,5 mm beträgt.

10. Verfahren nach einem der vorstehenden Ansprüche, wobei die Mikrogranulate mit mehreren Mikroschichten von Ambroxol-HCl und Filmbeschichtungskomponente beschichtet sind.

11. Pharmazeutische Ambroxol-HCl-Mikrogranulat-Darreichungsform, die Ambroxol-HCl gemäß einer vorgegebenen Steuerung abgibt, mit einer Anzahl von Kernmikrogranulaten mit verschiedenen Partikelgrößen im Bereich von 0,3-1,2 mm, die im wesentlichen kein pharmakologisch aktives Ambroxolhydrochlorid enthalten, wobei die Kernmikrogranulate mit einer Anzahl von Mikroschichten aus (a) mikrofiziertem Ambroxolhydrochlorid und (b) Filmmaterial mit gesteuerter Freigabe derart beschichtet sind, daß die beschichteten Mikrogranulate ihren Ambroxolhydrochloridanteil in vivo gemäß dem folgenden Freigaberatenprofil abgeben:
| | |
|---|---|
| 1. Stunde | 20% bis 40% |
| 8. Stunde | 55% bis 75% |
| 16. Stunde | > 75% |

12. Darreichungsform mit gesteuerter Freigabe nach Anspruch 11 mit 100 bis 500 mg mikrofiziertem Ambroxol-HCl pro Gramm der inerten Kernmikrogranulate.

13. Darreichungsform mit gesteuerter Freigabe nach Anspruch 11 oder 12, wobei die inerten Kernmikrogranulate Zucker und Stärke aufweisen.

14. Darreichungsform mit gesteuerter Freigabe nach einem der Ansprüche 11 bis 13, wobei das Filmmaterial mit verzögerter Freigabe Schellack, Polyvinylpyrrolidon oder Polyvinylpyrrolidon/Schellack-Mischung aufweist.

15. Darreichungsform mit gesteuerter Freigabe nach einem der Ansprüche 11 bis 14, wobei die äußere Mikroschicht des Filmmaterials mit verzögerter Freigabe Schellack, ein Akrylpolymer, Hydroxylpropylcellulose, Ethylcellulose oder Mischungen daraus aufweist.

16. Darreichungsform mit gesteuerter Freigabe nach einem der Ansprüche 11 bis 15, wobei die mehrfachen Mikroschichten aus mikrofiziertem Ambroxolhydrochlorid weiterhin Talk, Aluminium- oder Magnesiumsilikat, Laktose oder Stärke aufweisen.

17. Darreichungsform mit gesteuerter Freigabe nach einem der Ansprüche 1 bis 16 mit einer Außenbeschichtung aus einer Beschichtungskomponente, wobei die beschichteten Mikrogranulate Partikelgrößen im Bereich von 0,6-1,5 mm aufweisen.

18. Darreichungsform mit gesteuerter Freigabe nach einem der Ansprüche 1 bis 17, die ihren Ambroxolhydrochloridanteil in vivo so liefern kann, daß ein therapeutisch effektiver Plasmapegel von Ambroxol-HCl für mehr als 24 Stunden aufrechterhalten werden kann.

19. Darreichungsform mit gesteuerter Freigabe nach einem der Ansprüche 11 bis 18, wobei die externe Mikroschicht frei von dem aktiven Agens des mikrofizierten Ambroxolhydrochlorids ist.

20. Darreichungsform mit gesteuerter Freigabe nach einem der Ansprüche 11 bis 19, die in einer harten Gelatinekapsel aufgenommen ist, um einen unitären Dosisanteil von Ambroxol-HCl zu liefern.

## Revendications

1. Procédé de préparation de microgranules à libération programmée comprenant du chlorhydrate d'ambroxol, ledit procédé étant caractérisé en ce qu'il comprend les étapes suivantes :
formation de noyaux de 0,3 à 1,2 mm de diamètre ne contenant pratiquement aucune quantité pharmacologiquement active de chlorhydrate d'ambroxol,
enrobage desdits noyaux avec des microcouches multiples d'une composition comprenant un mélange de particules de chlorhydrate d'ambroxol de 1 à 150 micromètres de diamètre et d'un constituant d'enrobage par un film à libération retardée, ledit constituant d'enrobage étant tel que les microgranules aient une vitesse de libération de chlorhydrate d'ambroxol comme suit :
| | |
|---|---|
| première heure | 20-40 % |
| huitième heure | 55-75 % |
| seizième heure | > 75 % |

2. Procédé suivant la revendication 1, dans lequel les noyaux comprennent du saccharose, de l'amidon ou une association de saccharose et d'amidon.

3. Procédé suivant la revendication 1 ou la revendication 2, dans lequel au moins 50 % du constituant d'enrobage comprennent un ou plusieurs composés filmogènes qui sont insolubles dans un milieu aqueux, comprenant la gomme laque, une résine acrylique ou un mélange de ces composés.

4. Procédé suivant la revendication 3, dans lequel le constituant d'enrobage comprend 50 à 90 % du composé filmogène insoluble comprenant la gomme laque, une résine acrylique ou un mélange de ces composés.

5. Procédé suivant la revendication 4, dans lequel le constituant d'enrobage comprend 70 à 85 % du composé filmogène insoluble comprenant la gomme laque, une résine acrylique ou un mélange de ces composés.

6. Procédé suivant l'une quelconque des revendications 3, 4 et 5, dans lequel le reste du constituant d'enrobage comprend un composé hydrosoluble comprenant la polyvinylpyrrolidone, l'hydroxypropylméthylcellulose, l'éthylcellulose ou leurs dérivés.

7. Procédé suivant l'une quelconque des revendications précédentes, dans lequel le constituant d'enrobage comprend 75 à 80 % d'un composé filmogène insoluble dans l'eau comprenant la gomme laque et 15 à 25 % d'un composé filmogène hydrosoluble comprenant la polyvinylpyrrolidone.

8. Procédé suivant l'une quelconque des revendications précédentes, caractérisé en ce que les microgranules renferment du chlorhydrate d'ambroxol en une quantité de 100 à 500 mg/g de microgranules.

9. Procédé suivant l'une quelconque des revendications précédentes, caractérisé en ce que le diamètre des microgranules va de 0,6 à 1,5 mm.

10. Procédé suivant l'une quelconque des revendications précédentes, dans lequel les microgranules sont enrobés avec des microcouches multiples de ambroxol.HCl et de constituant d'enrobage par un film.

11. Forme posologique constituée de microgranules pharmaceutiques d'ambroxol.HCl qui libère l'ambroxol.HCl suivant un programme prédéterminé, comprenant une pluralité de microgranules centraux ayant divers diamètres de particules allant de 0,3 à 1,2 mm qui ne contiennent pratiquement aucune quantité pharmacologiquement active de chlorhydrate d'ambroxol, lesdits microgranules centraux étant enrobés avec des microcouches multiples (a) de chlorhydrate d'ambroxol micronisé et (b) d'une matière filmogène à libération retardée, de telle sorte que lesdits microgranules enrobés délivrent le chlorhydrate d'ambroxol qu'ils renferment, in vivo, suivant le profil de vitesse de libération suivant :
| | |
|---|---|
| première heure | 20-40 % |
| huitième heure | 55-75 % |
| seizième heure | > 75 % |

12. Forme posologique à libération programmée répondant à la définition suivant la revendication 11, comprenant 100 à 500 mg d'ambroxol.HCl micronisé par gramme des microgranules centraux inertes.

13. Forme posologique à libération programmée suivant la revendication 11 ou la revendication 12, dans laquelle les microgranules centraux inertes comprennent du sucre et de l'amidon.

14. Forme posologique à libération programmée suivant l'une quelconque des revendications 11 à 13, dans laquelle la matière filmogène à libération retardée comprend la gomme laque, la polyvinylpyrrolidone ou un mélange polyvinylpyrrolidone/gomme laque.

15. Forme posologique à libération programmée suivant l'une quelconque des revendications 11 à 14, dans laquelle la microcouche extérieure de matière filmogène à libération retardée comprend la gomme laque, un polymère acrylique, l'hydroxypropylcellulose, l'éthylcellulose ou un de leurs mélanges.

16. Forme posologique à libération programmée suivant l'une quelconque des revendications 11 à 15, dans laquelle les microcouches multiples de chlorhydrate d'ambroxol micronisé comprennent en outre du talc, du silicate d'aluminium ou de magnésium, du lactose ou de l'amidon.

17. Forme posologique à libération programmée suivant l'une quelconque des revendications 11 à 16, comprenant un enrobage extérieur de constituant d'enrobage, lesdits microgranules enrobés ayant des diamètres de particules allant de 0,6 à 1,5 mm.

18. Forme posologique à libération programmée suivant l'une quelconque des revendications 11 à 17, apte à délivrer le chlorhydrate d'ambroxol qu'elle renferme, in vivo, de manière à maintenir un taux plasmatique thérapeutiquement efficace d'ambroxol.HCl pendant un temps supérieur à 24 heures.

19. Forme posologique à libération programmée suivant l'une quelconque des revendications 11 à 18, dans laquelle la microcouche extérieure est dépourvue de l'agent actif consistant en chlorhydrate d'ambroxol micronisé.

20. Forme posologique à libération programmée suivant l'une quelconque des revendications 11 à 19, confinée à l'intérieur d'une capsule de gélatine dure pour fournir une dose unitaire de ambroxol.HCl.
